(19) Europäisches Patentamt
European Patent Office
Office européen des brevets

(11) EP 2 413 913 B1

(12) **EUROPEAN PATENT SPECIFICATION**

(45) Date of publication and mention of the grant of the patent:
**18.05.2022 Bulletin 2022/20**

(21) Application number: **10714386.9**

(22) Date of filing: **31.03.2010**

(51) International Patent Classification (IPC):
***A61K 9/16*** *(2006.01)* ***A61K 9/48*** *(2006.01)*
***A61K 31/4439*** *(2006.01)* ***A61P 1/00*** *(2006.01)*
***A61P 7/10*** *(2006.01)* ***A61P 9/12*** *(2006.01)*
***A61P 25/00*** *(2006.01)* ***A61P 25/14*** *(2006.01)*
***A61P 25/16*** *(2006.01)*

(52) Cooperative Patent Classification (CPC):
**A61K 9/1623; A61K 9/4858; A61K 31/4439; A61P 1/00; A61P 7/10; A61P 9/12; A61P 25/00; A61P 25/14; A61P 25/16**

(86) International application number:
**PCT/PT2010/000015**

(87) International publication number:
**WO 2010/114405 (07.10.2010 Gazette 2010/40)**

(54) **PHARMACEUTICAL FORMULATIONS COMPRISING NITROCATECHOL DERIVATIVES AND METHODS OF MAKING THE SAME**

PHARMAZEUTISCHE FORMULIERUNGEN MIT NITROCATECHOL-DERIVATEN UND HERSTELLUNGSVERFAHREN DAFÜR

FORMULATIONS PHARMACEUTIQUES CONTENANT DES DÉRIVÉS DE NITROCATÉCHOL ET PROCÉDÉS POUR LES PRÉPARER

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **01.04.2009 US 165794 P**

(43) Date of publication of application:
**08.02.2012 Bulletin 2012/06**

(73) Proprietor: **Bial-Portela & CA, S.A.**
**4745-457 S. Mamede do Coronado (PT)**

(72) Inventors:
- **VASCONCELOS, Teófilo Cardoso de**
**P-4745-457 S. Mamede do Coronado (PT)**
- **LIMA, Ricardo Jorge dos Santos**
**P-4745-457 S. Mamededo Coronado (PT)**
- **BARROCAS, Pedro Miguel da Costa**
**P-4745-457 S. Mamede do Coronado (PT)**
- **PEREIRA, Lígia Sofia de Castro**
**P-4745-457 S. Mamede do Coronado (PT)**
- **COSTA, Rui Cerdeira de Campos**
**P-4745-457 S. Mamede do Coronado (PT)**

(74) Representative: **Elkington and Fife LLP**
**Prospect House**
**8 Pembroke Road**
**Sevenoaks, Kent TN13 1XR (GB)**

(56) References cited:
**EP-A1- 1 845 097**
**WO-A1-2007/013830**
**WO-A1-2008/094053**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 413 913 B1

## Description

## FIELD OF THE DISCLOSURE

The present disclosure relates to compositions and pharmaceutical formulations comprising at least one active pharmaceutical ingredient chosen from nitrocatechol derivatives and salts thereof.

## BACKGROUND

Levodopa (L-DOPA) has been used in clinical practice for several decades in the symptomatic treatment of various conditions, including Parkinson's disease. L-DOPA is able to cross the blood-brain barrier, where it is then converted to dopamine and increases the levels thereof. However, conversion of L-DOPA to dopamine may also occur in the peripheral tissue, possibly causing adverse effects upon administration of L-DOPA. Therefore, it has become standard clinical practice to co-administer a peripheral amino acid decarboxylase (AADC) inhibitor, such as carbidopa or benserazide, which prevents conversion to dopamine in peripheral tissue.

This has led to an interest in the development of inhibitors of the enzyme catechol-O-methyltransferase (COMT) based on the hypothesis that inhibition of the enzyme may provide clinical improvements in patients afflicted with Parkinson's disease undergoing treatment with L-DOPA, since COMT catalyses the degradation of L-DOPA.

It has been found, as set forth in International Publication Nos. WO 2007/013830 and WO 2007/117165 that compounds of formula I disclosed herein, which are nitrocatechol derivatives, are potent and long-acting COMT inhibitors. Those compounds are both bioactive and bioavailable. Thus, compounds of formula I have potentially valuable pharmaceutical properties in the treatment of some central and peripheral nervous system disorders where inhibition of O-methylation of catecholamines may be of therapeutical benefit, such as, for example, mood disorders, Parkinson's disease and disorders, restless leg syndrome, gastrointestinal disturbances, oedema formation states, and hypertension. Furthermore, these compounds may also have activity in treating other diseases and disorders, not related to the inhibition of O-methylation of catecholamines. WO 2008/094053 A1 discloses that compounds of formula I disclosed herein can be administered according to a dosing regimen having a dosing periodicity ranging from about twice a day to about once every other day.

It has also been found, however, that the compounds of formula I are sensitive to certain excipients, which may cause decomposition of the compounds of formula I and/or lack of stability of the compositions and formulations containing these compounds. The compounds of formula I may also exhibit a low bulk density and/or poor flow characteristics, which may increase the difficulty in formulating and/or manufacturing a stable dosage formulation containing the active compound.

## SUMMARY

The inventors have now discovered stable compositions and formulations thereof comprising an active pharmaceutical ingredient ("API") chosen from a nitrocatechol derivative of formula I as defined herein and salts, esters, hydrates, and solvates thereof. The API which is a nitrocatechol derivative is 2,5-dichloro-3-(5-(3,4-dihydroxy-5-nitrophenyl)-1,2,4-oxadiazol-3-yl)-4,6-dimethylpyridine 1-oxide. The API is present in granular form and the composition has a bulk density greater than 0.5 g/mL. The nitrocatechol derivative may also be a mixture of the API 2,5-dichloro-3-(5-(3,4-dihydroxy-5-nitrophenyl)-1,2,4-oxadiazol-3-yl)-4,6-dimethylpyridine 1-oxide and further comprising 5-[3-(2,5-dichloro-4,6-dimethylpyridin-3-yl)-[1,2,4]oxadiazol-5-yl]-3-nitrobenzene-1,2-diol.

The API is present in granular form. In some embodiments, the compositions and/or formulations may comprise a further API, for example the compositions and/or formulations may comprise, in addition to the API which is a nitrocatechol derivative of formula I, further APIs such as L-DOPA, a peripheral amino acid decarboxylase (AADC) inhibitor, such as carbidopa or benserazide. The compositions and/or formulations also comprise at least one filler and at least one binder. The filler is not a phosphate derivative. The binder is not a polyvinylpyrrolidone ("PVP") derivative compound. In various embodiments when the API is present in granular form, the at least one filler and at least one binder may, independently, be intragranular (i.e., granulated with the API and/or contained within the same granules as the API), extragranular (i.e., present outside the granules of API), or part intragranular and part extragranular. The compositions exhibit a bulk density that is greater than that of the API alone (i.e., greater than 0.5 g/mL), and that may, in certain embodiments, be a significantly increased. The compositions may also exhibit improvements in other characteristics such as compressibility. Use of the methods described herein may also result in improvements in the granule properties of the compositions such as improved granule size and uniformity of granule size and/or of granule mass. The compositions and/or formulations are stable over time and under different conditions, and may, in certain embodiments exhibit enhanced stability.

## DETAILED DESCRIPTION

The present disclosure relates to stable compositions and formulations thereof comprising the API 2,5-dichloro-3-(5-(3,4-dihydroxy-5-nitrophenyl)-1,2,4-oxadiazol-3-yl)-4,6-dimethylpyridine 1-oxide which is a nitrocatechol derivative of formula I as defined herein and salts, esters, hydrates, and solvates thereof, at least one filler, and at least one binder. The at least one filler is not a phosphate derivative and the at least one binder is not a PVP derivative compound. The API is present in granular form and the composition has a bulk density greater than 0.5 g/mL.

As used herein, the term "granules," "granular form," "API granules" and variations thereof, refer to the particles produced by wet or dry granulation of the API which is a nitrocatechol derivative 2,5-dichloro-3-(5-(3,4-dihydroxy-5-nitrophenyl)-1,2,4-oxadiazol-3-yl)-4,6-dimethylpyridine 1-oxide of formula I as defined herein and salts, esters, hydrates, and solvates thereof. In various embodiments of the present disclosure, the API which is a nitrocatechol derivatives of formula I and is 2,5-dichloro-3-(5-(3,4-dihydroxy-5-nitrophenyl)-1,2,4-oxadiazol-3-yl)-4,6-dimethylpyridine 1-oxide further comprises 5-[3-(2,5-dichloro-4,6-dimethylpyridin-3-yl)-[1,2,4]oxadiazol-5-yl]-3-nitrobenzene-1,2-diol. The granules may further comprise at least one filler and/or at least one binder.

As used herein, the term "composition," and variations thereof, is intended to mean a composite comprising the API chosen from nitrocatechol derivative of formula I as defined herein and salts, esters, hydrates, and solvates thereof, at least one filler, and at least one binder. In certain embodiments, the composition may comprise two or more nitrocatechol derivatives of formula I, for example the composition may comprise the API 2,5-dichloro-3-(5-(3,4-dihydroxy-5-nitrophenyl)-1,2,4-oxadiazol-3-yl)-4,6-dimethylpyridine 1-oxide and 5-[3-(2,5-dichloro-4,6-dimethylpyridin-3-yl)-[1,2,4]oxadiazol-5-yl]-3-nitrobenzene-1,2-diol. The composition comprises granules of the API, and the at least one filler and at least one binder may independently be intragranular (i.e., granulated with the API and/or contained within the same granules as the API), extragranular (i.e., present outside the granules of API, or part intragranular and part extragranular. For example, the filler may be 10 wt% to 90 wt%, 20 wt% to 80 wt%, 30 wt% to 70 wt%, 40 wt% to 60 wt%, or about 50 wt% intragranular, with the remaining portion being extragranular. The binder may be 10 wt% to 90 wt%, 20 wt% to 80 wt%, 30 wt% to 70 wt%, 40 wt% to 60 wt%, or about 50 wt% intragranular, with the remaining portion being extragranular. The composition may further comprise at least one excipient, which may be intragranular, extragranular, or part intra- and part extra-granular. The composition is preferably suitable for filling a capsule, making a tablet, and/or for directly administering to patients, for example packaged in sachets.

As used herein, the terms "formulation," "pharmaceutical formulation," and variations thereof, are intended to include compositions described herein that are further processed or formulated into a dosage form. By way of example only, in various exemplary embodiments, the formulations may comprise a composition described herein, typically in the form of granules, in a dosage form suitable for administration to a subject, such as a capsule or compressed dosage form such as a tablet. In a further exemplary embodiment, the formulations may comprise a composition described herein, typically in the form of granules, mixed with at least one excipient in a dosage form suitable for administration to a subject, such as a capsule or compressed dosage form such as a tablet.

As used herein, the nitrocatechol derivatives of formula I are defined as follows:

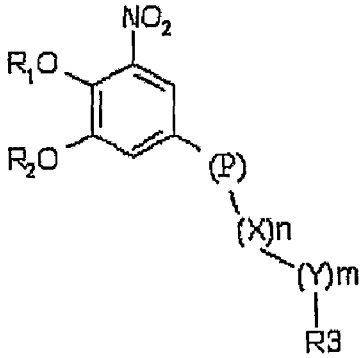

(I)

wherein:

- $R_1$ and $R_2$ are independently selected from hydrogen or a group which is hydrolysable under physiological conditions, optionally substituted lower alkanoyl or aroyl;
- X is a methylene group;
- Y is an atom of oxygen, nitrogen, or sulphur,
- n is selected from 0, 1, 2, and 3;

m is 0 or 1;

$R_3$ is a pyridine group chosen from the formulas A, B, C, D, E and F which is connected as indicated by the unmarked bond:

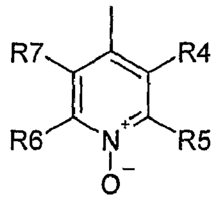
A

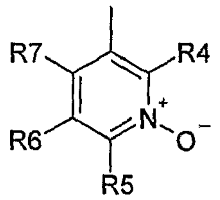
B

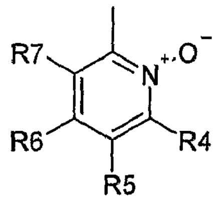
C

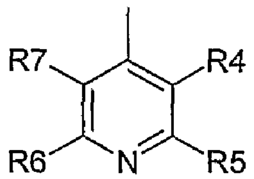
D

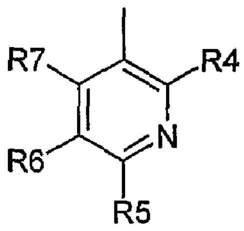
E

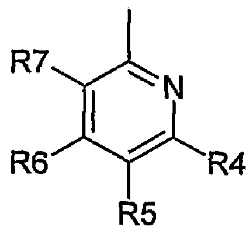
F

wherein:

$R_4$, $R_5$, $R_6$, and $R_7$ are independently chosen from hydrogen, $C_1$-$C_6$-alkyl, $C_1$-$C_6$-thioalkyl, $C_1$-$C_6$-alkoxy, $C_6$-$C_{12}$-aryloxy or a $C_6$-$C_{12}$-thioaryl group, $C_1$-$C_6$-alkanoyl or $C_7$-$C_{13}$-aroyl group, amino, $C_1$-$C_6$-alkylamino, $C_1$-$C_6$-dialkylamino, $C_3$-$C_{12}$-cycloalkylamino, $C_3$-$C_{12}$-heterocycloalkylamino, $C_1$-$C_6$-alkylsulphonyl, $C_6$-$C_{12}$-arylsulphonyl, halogen, $C_1$-$C_6$-haloalkyl, e.g., trifluoromethyl, cyano, nitro or a heteroaryl group; or two or more of residues $R_4$, $R_5$, $R_6$ and $R_7$ taken together represent aliphatic or heteroaliphatic rings or aromatic or heteroaromatic rings; and

P is a central unit, for example a planar unit, such as those selected from the regioisomers of 1,3,4-oxadiazol-2,5-diyl; 1,2,4-oxadiazol-3,5-diyl; 4-methyl-4H-1,2,4-triazol-3,5-diyl; 1,3,5-triazin-2,4-diyl; 1,2,4-triazin-3,5-diyl; 2H-tetrazol-2,5-diyl; 1,2,3-thiadiazol-4,5-diyl; 1-alkyl-3-(alkoxycarbonyl)-1H-pyrrol-2,5-diyl wherein alkyl is represented by methyl, ethyl, n-propyl and n-butyl and wherein alkoxy is represented by methoxy, ethoxy, n-propoxy and isopropoxy; 1-alkyl-1H-pyrrol-2,5-diyl wherein alkyl is represented by methyl, ethyl, n-propyl and n-butyl; thiazol-2,4-diyl; 1-H-pyrazol-1,5-diyl; pyrimidin-2,4-diyl; oxazol-2,4-diyl; carbonyl; 1H-imidazol-1,5-diyl; isoxazol-3,5-diyl; furan-2,4-diyl; 3-alkoxycarbonylfuran-2,4-diyl wherein alkoxy is represented by methoxy, ethoxy, n-propoxy, and isopropoxy; benzene-1,3-diyl; and (Z)-1-cyanoethen-1,2-diyl. Suitable groups which are hydrolysable under physiological conditions are well known in the art and include groups that form, with the O atom, an ether, ester, carbonic acid or an ester linkage.

Preferably, P is chosen from 1,3,4-oxadiazol-2,5-diyl and 1,2,4-oxadiazol-3,5-diyl.

The nitrocatechol derivative of formula I which is the API is 2,5-dichloro-3-(5-(3,4-dihydroxy-5-nitrophenyl)-1,2,4-oxadiazol-3-yl)-4,6-dimethylpyridine 1-oxide.

The at least one nitrocatechol derivative of formula I may also be a mixture of the API 2,5-dichloro-3-(5-(3,4-dihydroxy-5-nitrophenyl)-1,2,4-oxadiazol-3-yl)-4,6-dimethylpyridine 1-oxide and 5-[3-(2,5-dichloro-4,6-dimethylpyridin-3-yl)-[1,2,4]oxadiazol-5-yl]-3-nitrobenzene-1,2-diol.

In embodiments where the at least one nitrocatechol derivative is a mixture of two nitrocatechol derivatives, such as the API 2,5-dichloro-3-(5-(3,4-dihydroxy-5-nitrophenyl)-1,2,4-oxadiazol-3-yl)-4,6-dimethylpyridine 1-oxide and 5-[3-(2,5-dichloro-4,6-dimethylpyridin-3-yl)-[1,2,4]oxadiazol-5-yl]-3-nitrobenzene-1,2-diol, the ratio of the two components may be approximately 50:50 or any variation thereof, such as approximately 60:40, 70:30, 80:20, 90:10, 95:5, 97:3, or 99:1, or the proportion of one of the nitrocatechol derivatives may be present in an amount up to and including 5%, up to an including 3 % or up to and including 1% of the amount of the other nitrocatechol, for example 5-[3-(2,5-dichloro-4,6-dimethylpyridin-3-yl)-[1,2,4]oxadiazol-5-yl]-3-nitrobenzene-1,2-diol may be present in an amount of up to and including 5%, up to and including 3% or up to and including 1% of the amount of the API 2,5-dichloro-3-(5-(3,4-dihydroxy-5-nitrophenyl)-1,2,4-oxadiazol-3-yl)-4,6-dimethylpyridine 1-oxide.

The API chosen from nitrocatechol derivative 2,5-dichloro-3-(5-(3,4-dihydroxy-5-nitrophenyl)-1,2,4-oxadiazol-3-yl)-4,6-dimethylpyridine 1-oxide of formula I as disclosed herein, and salts, esters, hydrates, and solvates thereof, may exhibit low bulk density, thereby making it difficult to formulate and manufacture a dosage form. For example, 2,5-dichloro-3-(5-(3,4-dihydroxy-5-nitrophenyl)-1,2,4-oxadiazol-3-yl)-4,6-dimethylpyridine 1-oxide, the API nitrocatechol of formula I, exhibits a bulk density of less than 0.1 g/ml prior to granulation and/or formulation, and 5-[3-(2,5-dichloro-4,6-dimethylpyridin-3-yl)-[1,2,4]oxadiazol-5-yl]-3-nitrobenzene-1,2-diol may exhibit a bulk density of around 0.2 g/ml prior to granulation and/or formulation, as determined by the method described herein below.

Formulating APIs of low bulk density can often give rise to many problems. For example poor content uniformity, particle segregation, little or no flowability, high average weight variability, capping and lamination of tablets and high friability of tablets.

In at least one exemplary embodiment, the amount (or dosage) of the API present in the compositions and/or formulations is preferably a therapeutically effective amount. As used herein, "therapeutically effective amount" means an amount of a therapeutic agent sufficient to treat, alleviate, and/or prevent any condition treatable and/or preventable by administration of a composition of the disclosure, in any degree. That amount can, for example, be an amount sufficient to exhibit a detectable therapeutic or preventative or ameliorative effect. The effect may include, for example, treatment, alleviation, and/or prevention of the conditions listed herein. The actual amount required, e.g. for treatment of any particular patient, will depend upon a variety of factors including the disorder being treated and/or prevented; its severity; the specific pharmaceutical composition employed; the age, body weight, general health, gender, and diet of the patient; the mode of administration; the time of administration; the route of administration; the rate of excretion of the therapeutic agent; the duration of the treatment; any drugs used in combination or coincidental with the therapeutic agent; and other such factors well known to those skilled in the art. In various embodiments, for example, a formulation, i.e, a capsule or tablet dosage form, may contain 1 mg or more of API, for example 2.5 mg or more, 5 mg or more, 10 mg or more, 20 mg or more, 40 mg or more, 50 mg or more, or 100 mg or more of API. The API content in the formulation can vary from 0.02 wt% to 90 wt%, for example from 0.1 wt% to 70 wt%, from 0.2 wt% to 50 wt%, or from 0.3 wt% to 45 wt%.

The at least one filler of the present disclosure includes calcium carbonate, cellulose powder, silicified microcrystalline cellulose, cellulose acetate, compressible sugar, confectioner's sugar, dextrane, dextrin, dextrose, fructose, kaolin, lactitol, lactose, magnesium carbonate, magnesium oxide, maltodextrin, maltose, mannitol, microcrystalline cellulose, polydextrose, simethicone, sodium alginate, sodium chloride, sorbitol, starches, pregelatinized starch, sucrose, trehalose, and xylitol.

The at least one filler is not a phosphate derivative. As used herein, the term "phosphate derivative," and variations thereof, is intended to mean substances comprising calcium phosphate, including, but not limited to: calcium phosphate, dibasic anhydrous (for example, A-TAB ™, Di-Cafos A-N ™, Emcompress ™ Anhydrous, and Fujicalin ™); calcium phosphate, dibasic dihydrate (for example, Cafos ™, Calipharm ™, Calstar ™, Di-Cafos ™, Emcompress ™); and calcium phosphate tribasic (for example, Tri-Cafos ™, TRI-CAL ™ WG, TRI-TAB ™). In a further embodiment, the at least one filler may be chosen from starches, lactose, and cellulose. In at least one embodiment, at least two fillers may be present, for example a combination of starch, lactose, and/or cellulose.

In various embodiments, for example, the at least one filler may constitute 0.5 wt% to 99.5 wt% of the composition and/or formulation, for example, 20 wt% to 95 wt%, 40 wt % to 95 wt%, 40 wt % to 85 wt%, 40 wt % to 70 wt%, 60 wt% to 95 wt%, or 80 wt% to 95 wt% of the total weight of the composition and/or formulation. The filler may be intragranular, extragranular or part intragranular and part extragranular. By way of example, a composition and/or formulation may comprise 85 wt% filler. The amount of the at least one filler will vary depending, in part, upon the desired dosage, bulk density, and stability of the composition and/or formulation.

The at least one binder of the present disclosure may be selected from acacia, alginic acid, carbomer, carboxymethylcellulose sodium, ceratonia, cottonseed oil, dextrin, dextrose, gelatin, guar gum, hydrogenated vegetable oil type I, hydroxyethyl cellulose, hydroxyethylmethyl cellulose, hydroxypropyl cellulose, low substituted hydroxypropyl cellulose, hypromellose, magnesium aluminium silicate, maltodextrin, maltose, methylcellulose, ethylcellulose, microcrystalline cellulose, polydextrose, polyethylene oxide, polymethacrylates, sodium alginate, starch, pregelatinised starch, stearic acid, sucrose and zein.

The at least one binder is not a PVP derivative compound. As used herein, the term "PVP derivative compound" and variations thereof, is intended to mean substances comprising polyvinyl pyrrolidone (PVP) and substituted versions thereof, including, but not limited to: povidone (for example, plasdone and kollidon); copovidone (for example, plasdone S-630 ™ and kollidon VA-64 ™); and cross-linked PVP (for example crospovidone). In a further embodiment, the at least one binder may be chosen from starches, and in at least one embodiment, it may be starch 1500™.

In various embodiments, the at least one binder may constitute 0.5 wt% to 40 wt% of the composition and/or formulation, for example, 1 wt% to 25 wt%, 5 wt% to 20 wt%, 8 wt % to 15 wt%, or 10 wt% to 15 wt% of the total weight of the composition and/or formulation. The binder may be intragranular, extragranular or part intragranular and part extragranular. By way of example only, a composition and/or formulation may comprise between 6 wt% and 8 wt% binder, such as 7 wt% or 6.3 wt% binder. The amount of the at least one binder will vary depending, in part, upon the desired dosage, bulk density, and stability of the resulting composition and/or formulation.

In one exemplary embodiment, the composition and/or formulation comprises 0.2 to 50 wt% API, 5 to 10 wt% binder, and 33 to 85 wt% filler, such as the following compositions and/or formulations:

| | |
|---|---|
| API | 0.2 - 50 wt% |
| Filler | 35.0 - 85.0 wt% |
| Binder | 1.0 - 15.0 wt% |
| Lubricants | 1.0 - 15.0 wt% |
| Disintegrant | 1.0 - 15.0 wt % |

| | |
|---|---|
| API | 30.0-50.0 wt% |
| Filler | 35.0-60.0 wt% |
| Binder | 3.0-10.0 wt% |
| Lubricants | 1.0-10.0 wt% |
| Disintegrant | 3.0-10.0 wt% |

| | |
|---|---|
| API | 0.2 - 35 wt% |
| Filler | 50.0-85.0 wt% |
| Binder | 3.0-10.0 wt% |
| Lubricants | 1.0 - 10.0 wt% |
| Disintegrant | 3.0-10.0 wt% |

The invention also relates to formulations comprising a composition of the invention. Such formulations may be in the form of a dosage form such as a capsule or a compressed form such as a tablet.

The invention also includes a method of making a composition or formulation of the invention comprising the steps of:

- granulating the active pharmaceutical ingredient 2,5-dichloro-3-(5-(3,4-dihydroxy-5-nitrophenyl)-1,2,4-oxadiazol-3-yl)-4,6-dimethylpyridine 1-oxide and salts thereof to form granules;
- mixing at least one filler with the at least one active pharmaceutical ingredient before, during or after granulation;
- mixing at least one binder with the at least one active pharmaceutical ingredient before, during or after granulation; and
- preparing a pharmaceutical formulation in the form of a dosage form.

The filler is not a phosphate derivative. The binder is not a polyvinylpyrrolidone ("PVP") derivative compound.

The at least one API, at least one filler, and at least one binder may be combined by mixing (also referred to herein as blending). The appropriate apparatus and mixing time and rate may easily be determined by those of skill in the art based on, for example, the amount of material present, the type of mixing process used, and other parameters known to those of skill in the art. For example, in various embodiments, the components may be mixed manually, using a V-blender, a high shear mixer, or any other mixing apparatus and/or process known to those of skill in the art. As a further example, the components may be mixed for any appropriate period of time, such as 1 to 20 minutes or 2 to 10 minutes.

In various exemplary embodiments, the mixture may be dry or wet granulated. Preferably, the granules are wet-granulated using at least one granulation liquid. By way of example, the at least one granulation liquid may be chosen from water, ethanol, isopropanol, and/or acetone. The granulation liquid is preferably water. The appropriate apparatus and mixing time and rate for granulation may be determined by those of skill in the art based on, for example, the amount of material and the amount of granulation liquid, if present. For example, in various embodiments, the components may be granulated manually, using a high shear mixer, planetary mixer or any other granulator apparatus and/or process known to those of skill in the art. As a further example, in various embodiments, the components may be granulated for any appropriate period of time, such as 1 to 60 minutes or 2 to 30 minutes. Determination of the endpoint of granulation is within the capability of the skilled person but can be determined by observance of stabilization of granule size and particle cohesion resulting in a decrease in air trapped inside the granule, or by attainment of steady state of rheological or correlated determination of voltage, conductivity torque, power consumption or near IR techniques. Granulation speeds may vary from 5% to 100% of the granulator mixing speed, such as from 25% to 100%.

After the wet-granulation process is complete, the granules may then be dried. Granules may be dried to loss

on drying (LOD) values below 6%, preferably below 5% and even more preferably between 1-3%. A suitable method for calculating LOD is described hereinbelow. The appropriate drying apparatus and drying time and temperature may be determined by those of skill in the art based on, for example, the amount of material present, moisture content of the material, and the granulation liquid. As non-limiting examples, a fluid bed dryer or tray dryer may be used, for example at a temperature of 25°C or higher, 40°C or higher, or 70°C or higher, to dry the granules. For example, the granules may be dried at a temperature of 66°C.

The granules may be sieved. Sieving the granules separates out granules of a particular particle size, and may be used to select particles of an advantageous size for formulating a dosage form or manufacturing a dosage form. In various embodiments, the granules may be sieved over a screen or sieve of 0.5 mm or larger, for example a 0.6 mm, 0.8 mm, 1.0 mm and 1.6 mm screen.

The composition may further include at least one additional excipient which may be blended with the at least one API, at least one filler and at least one binder before, during or after granulation. For example, in at least one embodiment, the at least one additional excipient may be chosen from excipients such as disintegrants, glidants, and lubricants.

Suitable disintegrants of the present disclosure include agar, calcium carbonate, alginic acid, calcium phosphate (tribasic), carboxymethylcellulose calcium, carboxymethylcellulose sodium, colloidal silicon dioxide, croscarmellose sodium, crospovidone, docusate sodium, guar gum, low substituted hydroxypropyl cellulose, magnesium aluminium silicate, methylcellulose, microcrystalline cellulose, sodium alginate, sodium starch glycolate, polacrilin potassium, silicified microcrystalline cellulose, starch and pre- gelatinized starch, and mixtures thereof. The disintegrant may be a combination of disintegrants and/or at least two disintegrants are present, for example a combination of sodium starch glycolate and sodium carboxymethyl starch, such as that sold under the trade name Explotab™.

The disintegrant may constitute 0.5 wt% to 40 wt% of the composition and/or formulation, for example, 1 wt% to 25 wt%, 5 wt% to 20 wt%, 10 wt % to 15 wt%, or 5 wt% to 15 wt%. By way of example, a composition and/or formulation may comprise between 6 wt% and 9 wt% disintegrant, such as 6.8 wt% disintegrant. The amount of the at least one disintegrant will vary depending, in part, upon the desired dosage, bulk density, and stability of the resulting composition and/or formulation.

Suitable glidants of the present disclosure include calcium silicate, cellulose, powdered, colloidal silicon dioxide, magnesium silicate, magnesium trisilicate, starch, and talc, and mixtures thereof.

The glidant may constitute 0.1 wt% to 15 wt% of the composition and/or formulation, for example, 0.5 wt% to 15 wt%, 1 wt % to 10 wt%, or 2 wt% to 6 wt%. The amount of glidant will vary depending, in part, upon the desired dosage, bulk density, and stability of the resulting composition and/or formulation.

Lubricants of the present disclosure include calcium stearate, glycerine monostearate, glyceryl behenate, glyceryl palmitostearate, hydrogenated castor oil, hydrogenated vegetable oil type I, magnesium lauryl sulphate, magnesium stearate, medium-chain triglycerides, poloxamer, polyethylene glycol, sodium benzoate, sodium chloride, sodium lauryl sulphate, sodium stearyl fumarate, stearic acid, talc, sucrose stearate, and zinc stearate, and mixtures thereof.

Lubricants may constitute 0.1 wt% to 15 wt% of the composition and/or formulation, for example, 0.5 wt% to 15 wt%, 1 wt % to 10 wt%, 1 wt % to 2 wt%, or 2 wt% to 8 wt%. The amount of lubricant will vary depending, in part, upon the desired dosage, bulk density, and stability of the resulting composition and/or formulation.

The at least one excipient may be added before, during or after mixing of the at least one API and before (prior to) or during granulation and, thus may be an intragranular excipient. Alternatively, the at least one excipient may be added to the composition after granulation, for example by blending with the granules, and thus may be present as an extragranular excipient. In various embodiments, at least one first excipient may be added prior to or during granulation and at least one second excipient and/or more of the at least one first excipient may be added to the composition after granulation. For example, disintegrants may be added prior to or during granulation, whereas lubricants and glidants may be added after granulation.

The composition comprising at least one API, at least one filler, and at least one binder may be used to make a formulation, such as, for example, to fill capsules or to form tablets.

Capsules for use in the present disclosure include, but are not limited to, gelatin capsules and hydroxypropylmethyl cellulose (hypromellose) capsules. Suitable methods for filling such capsules with a composition according to an embodiment of the disclosure are well-known to those of skill in the art.

Tablets of the present disclosure may be formed by any method known to those of skill in the art such as compression. In at least one embodiment of the present disclosure, the tablets may be coated, for example with aqueous based film-coatings, solvent based film-coatings and/or sugar coatings.

The formulations of the invention may also be coloured, for example by inclusion of a colouring in the composition of the invention and/or by coating the composition and/or formulation.

In at least one embodiment of the present disclosure, the formulation is a capsule comprising at least one API, at least one filler, and at least one binder, optionally in granular form, and may further comprise at least one glidant and/or at least one disintegrant. In at least one embodiment of the present disclosure, the formulation is a tablet comprising

at least one API, at least one filler, and at least one binder, optionally in granular form, and may further comprise at least one glidant, at least one lubricant, and/or at least one disintegrant.

The compositions may exhibit improved bulk density and/or flow properties relative to those of the API alone. As used herein, the terms "improved bulk density," "significantly improved bulk density," and variations thereof mean that the bulk density of the composition is approximately at least double, least three times, at least four times, or at least five times that of the API alone. It is within the ability of one of skill in the art to determine the bulk density of a compound or composition using methods generally accepted in the art. However, suitable methods include, for example, the European Pharmacopeia edition 6, Test 2.9.15 "apparent volume," pages 285-286, EDQM, 2007, and USP 31, vol. 1, test <616> page 231-232, The United States Pharmacopeia Convention, 2008. A suitable method is described below:

Apparatus:

- settling apparatus capable of producing in 1 minute 250 + 15 taps from a height of 3 ± 0.2 mm. The support for the graduated cylinder with its holder, has a mass of 450 + 5 g
- a 250 ml graduated cylinder (2 ml intervals) with a mass of 220 + 40 g

Method: Into a dry cylinder, introduce without compacting, 100.0 g (m g) of the test substance. Secure the cylinder in its holder. Read the unsettled apparent volume (Vo) to the nearest milliliter. Carry out 10, 500 and 1250 taps and read the corresponding volumes $V_{10}$, $V_{500}$, $V_{1250}$, to the nearest milliliter. If the difference between $V_{500}$ and $V_{1250}$ is greater than 2 ml, carry out another 1250 taps.

Alternatively, if it is not possible to select 100.0 g, select a test sample of any mass but with a volume between 50 ml and 250 ml, measure its apparent volume, $V_0$ as described above, and weigh the sample and specify the mass in the expression of results. Bulk/apparent density may then be determined in g/ml using the following formula:

$$m/V_0$$

where *m* is the mass in grams and $V_0$ the unsettled apparent volume.

Tapped apparent density may then be determined in g/ml using the following formula:

$$M/V_{1250}$$

where m is the mass in grams and $V_{1250}$ the apparent volume after 1250 hubs.

For example, as set forth above, the API 2,5-dichloro-3-(5-(3,4-dihydroxy-5-nitrophenyl)-1,2,4-oxadiazol-3-yl)-4,6-dimethylpyridine 1-oxide, a nitrocatecol of formula I, exhibits a bulk density of less than 0.1 g/ml prior to granulating. Compositions according to the present disclosure comprising the API 2,5-dichloro-3-(5-(3,4-dihydroxy-5-nitrophenyl)-1,2,4-oxadiazol-3-yl)-4,6-dimethylpyridine 1-oxide exhibit bulk densities of 0.5 g/ml or greater. Compositions of the present disclosure for use as final blends for capsule filling or tabletting comprising the API 2,5-dichloro-3-(5-(3,4-dihydroxy-5-nitrophenyl)-1,2,4-oxadiazol-3-yl)-4,6-dimethylpyridine 1-oxide may also exhibit bulk densities of 0.5 g/ml or greater, and 0.6 /ml or greater.

In certain embodiments of the disclosure, compressed formulations of the disclosure, such as tablets, exhibit apparent density of 0.5 g/mL to 1.5 g/mL, such as 0.6 g/mL to 1.4 g/mL, 0.7 g/mL to 1.3 g/mL, or 0.8 g/mL to 1.2 g/mL.

The apparent density of a compressed formulation is measured in terms of mass and volume of the formulation and is well within the capabilities of the skilled person.

It is also within the ability of one of skill in the art to determine the flowability/flow rate of a compound or composition using methods generally accepted in the art. However, suitable methods include, for example, testing the flow rate through an orifice described in USP 31, vol. 1, test <1174>, The United States Pharmacopeia Convention, 2008. The flowability may be measured as the mass per time flowing through the 10 mm diameter opening of a glass funnel. A flow rate of value greater than 10 g/second is considered good whereas a value of less than 10 g/second is considered poor.

The compressibility index and Hausner ratio are also suitable methods to assess the compound or compositions. For example, the compressibility index and Hausner ratio may be assessed using USP 31, vol. 1, test <1174>, The United States Pharmacopeia Convention, 2008, and measuring both the bulk volume (Vo) and the tapped volume ($V_f$) of the granules. The compressibility index (CI) may then be calculated using the following formula:

$$CI\ (\%) = 100 \times [(V_0 - V_f)/V_0]$$

The Hausner ratio (HR) can be calculated by using the following formula:

$$HR = V_0/V_f$$

A compressibility index is considered good when a value of less than 15% is calculated. A Hausner ratio value (a measure of flowability) is considered good when a value of less than 1.25 is calculated.

The compositions and/or formulations are stable and/or exhibit enhanced stability over other compositions and/or formulations. As used herein, the terms "stability," "stable," and variations thereof, is intended to mean that less than 15 wt% of the at least one API in the composition and/or formulation decomposes over 6 months at test conditions of 40°C and 75% relative humidity, or over 3 years at test conditions of 25°C or 30°C and 60% relative humidity or over 15-30 days at test conditions of 70°C and uncontrolled humidity. In various embodiments, for example, less than 10 wt%, less than 8 wt%, less than 6 wt%, less than 5 wt%, less than 4 wt%, less than 3 wt%, less than 2 wt%, or less than 1 wt% of the at least one API may decompose under these conditions. It is within the ability of one of skill in the art to determine the stability of a compound, composition, or formulation using methods generally accepted in the art. For example, the amount of the at least one API may be measured by any suitable method, e.g., HPLC. For example, in various embodiments, the assay (i.e. the amount of API) of a stable composition or formulation may indicate 85-115% of API after testing conditions, such as 95-105% of API.

Decomposition is a chemical process made up of at least one reaction, such as oxidation, reduction or hydrolysis, that results in a chemical change in the decomposing substance resulting in the generation of one or more new chemical compounds. These new compounds (or impurities) may result in reduced and/or variable amount of the API in a given composition and/or formulation, reducing its efficacy, and may have unwanted and/or harmful side effects on the patients. As used herein the term "impurity" means any such new compound that is present in the composition and/or formulation in an amount less than 10 wt% of the API, for example less than 5 wt%, less than 3 wt%, less than 1wt %, or less than 0.5 wt% of the API. Thus, the change in total impurities in the composition and/or formulation under the conditions and time periods set forth herein may also be indicative of a stable composition or formulation and may be measures by a suitable method, e.g., HPLC. In various embodiments, for example, the total impurities relative to the API in a stable composition and/or formulation after testing conditions may increase by less than 5 wt%, less than 2 wt%, less than 1 wt% or less than 0.5 wt%,

Stability may also be tested under the influence of a variety of other test conditions, including, for example:

- 40°C at 75% relative humidity for 6 months;
- 25°C or 30°C at 60% relative humidity after 3-5 years (long-term conditions); and
- 70°C at uncontrolled humidity after 15-30 days (stress conditions).

Stability may also be determined by appearance. As used herein, the term "visual stability," and variations thereof, is intended to mean insubstantial changes in the color, integrity of a compressed formulation (for example, not breaking up), shape, and/or size of the granules, composition and/or formulation.

As used herein, the term "enhanced stability," "improved stability" and variations thereof, means that the amount of decomposition of the at least one API in a given composition and/or formulation, and/or the increase in impurities in a given composition and/or formulation is less than that of a comparative composition and/or formulation that has been subject to the test conditions.

Unless otherwise indicated, all numbers used in the specification and claims are to be understood as being modified in all instances by the term "about," whether or not so stated. It should also be understood that the precise numerical values used in the specification and claims form additional embodiments of the disclosure. Efforts have been made to ensure the accuracy of the numerical values disclosed in the Examples. Any measured numerical value, however, can inherently contain certain errors resulting from the standard deviation found in its respective measuring technique.

As used herein the use of "the," "a," or "an" means "at least one," and should not be limited to "only one" unless explicitly indicated to the contrary. Thus, for example, the use of "the formulation" or "a formulation" is intended to mean at least one formulation.

## EXAMPLES

### Example 1

Four low dosage capsules were made on a pilot batch scale by first mixing the API, starches, and lactose in the amounts set forth in Table 1 below (batches A-D). The API used in these examples was 2,5-dichloro-3-(5-(3,4-dihydroxy-5-nitrophenyl)-1,2,4-oxadiazol-3-yl)-4,6-dimethylpyridine 1-oxide. Purified water was then added to each mixture, and the mixtures were granulated by mixing.

The granules were then dried using a fluid bed dryer until a loss on drying value of the granule was below 6%. The dried granules were sieved and then blended with the remaining ingredients set forth in Table 1. Gelatin capsules were filled with the formulation using an InCAP HS capsule filling machine.

The granules and final compositions were evaluated for bulk and tapped density using the methods described above. Flowability/flow rate was also assessed by testing the flow rate through an orifice described in USP 31, vol. 1, test <1174>, The United States Pharmacopeia Convention, 2008. The flowability was measured as the mass per time flowing through the 10 mm diameter opening of a glass funnel. A flow rate of value greater than 10 g/second is considered good whereas a value of less than 10 g/second is considered poor.

The compressibility index and Hausner ratio were assessed using USP 31, vol. 1, test <1174>, The United States Pharmacopeia Convention, 2008, and measuring both the bulk volume (Vo) and the tapped volume ($V_f$) of the granules. The compressibility index (CI) was then calculated using the following formula:

$$CI\ (\%) = 100 \times [(V_0-V_f)/V_0]$$

The Hausner ratio (HR) can be calculated by using the following formula:

$$HR = V_0/V_f$$

A compressibility index is considered good when a value of less than 15% is calculated. A Hausner ratio value (a measure of flowability) is considered good when a value of less than 1.25 is calculated.

Moisture or dryness was determined by loss on drying as described in USP 31, vol. 1, test <731>, The United States Pharmacopeia Convention, 2008. The test involves accurately weighing the substance to be tested (mo), (e.g. using a sample amount of 1 to 2 g). The test specimen is then dried at 105°C until a constant weight ($m_f$) is achieved. The moisture can be calculated by using the following expression:

$$LOD\ (\%) = [(m_o-m_f)/m_0] * 100$$

Capsules were evaluated for uniformity of mass and impurities. Uniformity of mass was assessed by the individual weight of 20 capsules; average mass and standard deviation were then calculated. Amount of total impurities was obtained using HPLC method with a limit of quantification of below 0.05%.

The results are set forth in Table 2 below. All batches presented good granule and capsule properties.

Example 2

Four high dosage capsules were made on a laboratory scale by first mixing the API, starches, and lactose in the amounts set forth in Table 1 below (batches E-H) in a V-blender. The API used in these examples was 2,5-dichloro-3-(5-(3,4-dihydroxy-5-nitrophenyl)-1,2,4-oxadiazol-3-yl)-4,6-dimethylpyridine 1-oxide. Purified water was added to each mixture and mixed manually. The wet mass thus obtained was then granulated in an oscillation granulator laboratory.

The granules were then dried in a tray dryer until a loss on drying of the granule was below 6%. The dried granules were sieved. The granules were then blended with the remaining ingredients set forth in Table 1 in a V- blender. Gelatin capsules were filled with the formulation using an InCAP HS capsule filling machine.

Each of Batch E-H was evaluated as set forth in Example 1 above and the results are set forth in Table 3 below. All batches presented good granule and capsule properties.

Table 1: Batch Formulations

| Ingredient (%/capsule) | BATCH | | | | | | | |
|---|---|---|---|---|---|---|---|---|
| | A | B | C | D | E | F | G | H |
| API 9-1067 | 0.4 | 0.4 | 0.4 | 0.4 | 40 | 40 | 40 | 40 |
| Maize starch | 0 | 82.0 | 40.8 | 26.0 | 0 | 42.4 | 21.2 | 14.0 |
| Lactose 200 | 82.0 | 0 | 41.2 | 56.0 | 42.4 | 0 | 21.2 | 28.4 |
| Starch 1500™ | 6.8 | 6.8 | 6.8 | 6.8 | 6.8 | 6.8 | 6.8 | 6.8 |

(continued)

| Ingredient (%/capsule) | BATCH | | | | | | | |
|---|---|---|---|---|---|---|---|---|
| | A | B | C | D | E | F | G | H |
| Explotab ™ | 6.8 | 6.8 | 6.8 | 6.8 | 6.8 | 6.8 | 6.8 | 6.8 |
| Purified water | q.ad | q.ad | q.ad | q.ad | q.ad | q.ad | q.ad | q.ad |
| Silica colloidal hydrated (Syloid™) | 2.0 | 2.0 | 2.0 | 2.0 | 2.0 | 2.0 | 2.0 | 2.0 |
| Talc | 2.0 | 2.0 | 2.0 | 2.0 | 2.0 | 2.0 | 2.0 | 2.0 |
| capsule size 1 | 1 un | 1 un | 1 un | 1 un | 1 un | 1 un | 1 un | 1 un |

Table 2: Analytical Results for Batches A-D

| **BATCH** | **A** | **B** | **C** | **D** |
|---|---|---|---|---|
| Granule results | | | | |
| Bulk density (g/ml) | 0.62 | 0.59 | 0.60 | 0.64 |
| Tapped density 1250 (g/ml) | 0.75 | 0.73 | 0.70 | 0.74 |
| Compressibility index | Good | Good | Good | Good |
| Hausner ratio | Good | Good | Good | Good |
| Flow rate | Good | Good | Good | Good |
| | | | | |
| Capsule results | | | | |
| Uniformity mass (RSD %) | 2.39 | 1.77 | 1.26 | 1.14 |
| Impurities (%) | 3.34 | 3.30 | 3.23 | 3.38 |

Table 3: Analytical Results for Batches E-H

| **BATCH** | **E** | **F** | **G** | **H** |
|---|---|---|---|---|
| Granule results | | | | |
| Bulk density (g/ml) | 0.60 | 0.57 | 0.55 | 0.56 |
| Tapped density 1250 (g/ml) | 0.68 | 0.62 | 0.60 | 0.64 |
| Compressibility index | Good | Good | Good | Good |
| Hausner Ratio | Good | Good | Good | Good |
| Flow rate | Good | Poor | Poor | Poor |
| Capsule results | | | | |
| Uniformity mass (RSD %) | 2.86 | 2.34 | 2.08 | 2.89 |
| Impurities (%) | 2.95 | 3.07 | 2.98 | 2.97 |

Example 3

All batches from Examples 1 and 2 were put on stress studies to determine their stability. Each of the eight batches was stored for 15 days at room temperature as well as under stress conditions (70°C without relative humidity control). All batches were tested for impurities content for both storage conditions, the results of which are set forth in Tables 4 and 5. Impurities values were obtained using HPLC method with a limit of quantification of below 0.05%.

API used in these batches contained around 3% of impurities prior to formulation (composed of impurity 8).

Table 4: Results from Stability Testing on Batches A-D

| | A | | B | | c | | D | |
|---|---|---|---|---|---|---|---|---|
| Dosage | **Low** | | **Low** | | **Low** | | **Low** | |
| Storage | **RT** | **SC** | **RT** | **SC** | **RT** | **SC** | **RT** | **SC** |
| **Total Impurities (%)** | **3.34** | **3.89** | **3.30** | **5.04** | **3.23** | **5.36** | **3.39** | **4.03** |
| Impurity 8 | 3.34 | 3.03 | 3.30 | 2.90 | 3.23 | 3.29 | 3.39 | 3.17 |
| Impurity 1 | <0.05 | 0.76 | <0.05 | 1.37 | NP | 1.38 | NP | 0.68 |
| Impurity 2 | NP | 0.06 | NP | 0.05 | NP | 0.19 | NP | 0.11 |
| Impurity 3 | NP | NP | NP | 0.07 | NP | 0.14 | NP | NP |
| Impurity 4 | NP | NP | NP | 0.20 | NP | 0.10 | <0.05 | <0.05 |
| Impurity 5 | NP | NP | NP | 0.18 | NP | 0.15 | NP | NP |
| Impurity 6 | NP | 0.07 | NP | 0.14 | NP | 0.14 | NP | 0.07 |
| Impurity 7 | NP | <0.05 | NP | 0.12 | NP | 0.13 | NP | NP |
| RT - room temperature<br>SC - Stress conditions<br>NP- not present (below detection limit) | | | | | | | | |

Table 5: Results from Stability Testing on Batches E-H

| | E | | F | | G | | H | |
|---|---|---|---|---|---|---|---|---|
| Dosage | High | | High | | High | | High | |
| Storage | **RT** | **SC** | **RT** | **SC** | **RT** | **SC** | **RT** | **SC** |
| **Total Impurities (%)** | **2.96** | **2.86** | **3.07** | **3.03** | **2.98** | **2.78** | **2.97** | **2.78** |
| Impurity 8 | 2.96 | 2.78 | 3.07 | 2.94 | 2.98 | 2.71 | 2.97 | 2.75 |
| Impurity 1 | 0.06 | 0.08 | NP | 0.10 | NP | 0.07 | NP | 0.06 |
| Impurity 2 | NP | <0.05 | NP | <0.05 | NP | NP | NP | NP |
| Impurity 3 | NP | NP | NP | NP | NP | NP | NP | NP |
| Impurity 4 | <0.05 | <0.05 | <0.05 | <0.05 | NP | NP | NP | NP |
| Impurity 5 | NP | NP | NP | NP | NP | NP | NP | NP |
| Impurity 6 | NP | NP | NP | NP | NP | NP | NP | NP |
| Impurity 7 | NP | NP | NP | NP | NP | NP | NP | NP |
| RT - room temperature<br>SC - Stress conditions<br>NP- not present (below detection limit) | | | | | | | | |

Example 4

Batch E from Example 2 was put on long term stability studies to determine its stability. In one study, the batch was stored for 6 months at 25°C and 60% relative humidity, and in a second study, the batch was stored for 6 months at 40°C and 75% relative humidity. After each test, the batch was tested for assay and impurities content, the results of which are set forth in Table 6. Assay and impurities values were obtained using HPLC method with a limit of quantification of below 0.05%.

Table 6: Stability Data for Batch E

| **Batch** | **E** | | |
|---|---|---|---|
| **Time** | 0 | 6 months | 6 months |
| **Storage** | | 25°C / 60% RH | 40°C / 75% RH |
| **Assay (%)** | **96** | **99** | **98** |
| **Change in Total Impurities Content(%)** | **<0.05** | **<0.05** | **<0.05** |

Comparative Example

Three intermediate dosage capsules were made by first mixing the API, the filler(s) the binder and the disintegrant (smaller portion in comparative example and the total amount in batches I and J) in the amounts set forth in Table 7 below for 3 minutes in a high shear mixer. The API used in these examples was 2,5-dichloro-3-(5-(3,4-dihydroxy-5-nitrophenyl)-1,2,4-oxadiazol-3-yl)-4,6-dimethylpyridine 1-oxide. Purified water was added to each mixture over a 3 minute period, and the mixtures were granulated by mixing for an additional 3 minutes.

The granules were then dried in a fluid bed dryer until a loss on drying value of the granule was below 6%. The dried granules were sieved and then blended with the remaining ingredients set forth in Table 7 in a biconic blender. Gelatin capsules were filled with the formulation using an InCAP HS capsule filling machine.

Table 7: Formulations for Comparative Example 1

| **BATCH** | **Comp.** | **I** | **J** |
|---|---|---|---|
| Ingredient (%/capsule) | | | |
| API | 2 | 2 | 2 |
| Di-Calcium-Phosphate (filler) | 33 | | |
| Lactose(filler) | | 80 | 54 |
| Microcrystalline Cellulose(filler) | 46 | | |
| Croscarmellose- Sodium (disintegrant) | 2 | | |
| Maize starch (filler) | | | 27 |
| Povidone (binder) | 7 | | |
| Purified Water | q.s. | q.ad | q. ad |
| Modified starch (Binder) | | 7 | 7 |
| Sodium amidoglycolate (Disintegrant) | | 7 | 7 |
| Croscarmellose- Sodium (disintegrant) | 4 | | |
| Silica Colloidal Hydrate (lubricant) | 4 | 2 | 2 |
| Talc (lubricant) | 2 | 2 | 2 |
| Magnesium-Stearate (lubricant) | 2 | | |

The granules and capsules were evaluated and results are shown in Table 8 below. After two stability studies, one under 25°C and 60% RH and the other under 40°C and 75% RH for 6 months each, it was observed that batches I and J exhibit enhanced stability when compared with the comparative composition.

Table 8: Stability of Formulae after 6 Months at 40°C at 75% RH

| **Batch** | Comparative (Comp.) | | | I | | | J | | |
|---|---|---|---|---|---|---|---|---|---|
| **Time (months)** | 0 | 6 | 6 | 0 | 6 | 6 | 0 | 6 | 6 |
| **Storage** | | 25°C / 60% RH | 40°C / 75% RH | | 25°C / 60% RH | 40°C / 75% RH | | 25°C / 60% RH | 40°C / 75% RH |
| **Assay (%)** | 99 | 99 | 92 | 98 | 100 | 102 | 97 | 98 | 100 |

(continued)

| **Batch** | Comparative (Comp.) | | | I | | | J | | |
|---|---|---|---|---|---|---|---|---|---|
| **Change in Total Impurities Content** | | 0.07 | 2.34 | | None detected | 0.15 | | None detected | 0.15 |

## Claims

1. A stable composition comprising:

   an active pharmaceutical ingredient (API) chosen from 2,5-dichloro-3-(5-(3,4-dihydroxy-5-nitrophenyl)-1,2,4-oxadiazol-3-yl)-4,6-dimethylpyridine 1-oxide and salts, esters, hydrates, and solvates thereof;
   at least one filler; and
   at least one binder;
   wherein the active pharmaceutical ingredient is present in granular form;
   wherein the at least one filler is not a phosphate compound;
   wherein the at least one binder is not a PVP compound; and
   wherein the composition has a bulk density greater than 0.5 g/mL.

2. The stable composition of claim 1, further comprising 5-[3-(2,5-dichloro-4,6-dimethylpyridin-3-yl)-[1,2,4]oxadiazol-5-yl]-3-nitrobenzene-1,2-diol.

3. The stable composition of claims 1 or 2, wherein:

   a. less than 10% or less than 3% of the API decomposes:

      i. over 15 days of storage at 70°C and uncontrolled humidity;
      ii. over 6 months at 40°C and 75% relative humidity; and/or
      iii. over 3 years at 60% relative humidity and 30°C or 25°C; and/or

   b. the increase in total impurities is less than 5% or less than 0.5%:

      i. over 15 days of storage at 70°C and uncontrolled humidity;
      ii. over 6 months at 40°C and 75% relative humidity; and/or
      iii. over 3 years at 60% relative humidity and 30°C or 25°C.

4. The stable composition of any preceding claim wherein the at least one filler is chosen from lactose, maize starch and microcrystalline cellulose and wherein the at least one binder is chosen from hypromellose, hydroxypropyl cellulose, methyl- or ethyl-cellulose, pregelatinized maize starch and gelatin.

5. The stable composition of any preceding claim:

   a. wherein the granules further comprise the at least one filler and/or at least one binder; or
   b. further comprising at least one additional excipient chosen from disintegrants, glidants, and lubricants; or
   c. wherein the composition exhibits a bulk density of greater than 0.6 g/ml; or
   d. wherein the composition exhibits an apparent density greater than 0.1 g/ml, or greater than 0.5 g/ml.

6. The stable composition of claim 5, wherein the lubricant is selected from glycerine monostearate, glyceryl behenate, glyceryl palmitostearate, hydrogenated castor oil, hydrogenated vegetable oil type I, medium-chain triglycerides, calcium stearate, magnesium lauryl sulphate, magnesium stearate, sodium lauryl sulphate, sodium stearyl fumarate, stearic acid, and zinc stearate, preferably calcium stearate, magnesium lauryl sulphate, magnesium stearate, sodium lauryl sulphate, sodium stearyl fumarate, stearic acid, and zinc stearate.

7. A pharmaceutical formulation comprising the stable composition of any preceding claim.

**8.** The pharmaceutical formulation of claim 7, wherein

a. the active pharmaceutical ingredient is present in a therapeutically effective amount; or
b. the composition comprises an additional API; or
c. the formulation is in the dosage form of a tablet or capsule; or
d. the pharmaceutical formulation is stable.

**9.** The pharmaceutical formulation according to claim 7 or claim 8, wherein the API is present in an amount of 1 mg or more, 2.5 mg or more, 5 mg or more, 10 mg or more, 20 mg or more, 40 mg or more, 50 mg or more, or 100 mg or more and wherein the at least one API is present in granular form.

**10.** The pharmaceutical formulation according to claim 9, further comprising carbidopa or benserazide.

**11.** A method of manufacturing a stable pharmaceutical formulation, said method comprising:

granulating an active pharmaceutical ingredient (API) chosen from 2,5-dichloro-3-(5-(3,4-dihydroxy-5-nitrophenyl)-1,2,4-oxadiazol-3-yl)-4,6-dimethylpyridine 1-oxide and salts thereof to form granules;
mixing at least one filler with the at least one active pharmaceutical ingredient before, during or after granulation;
mixing at least one binder with the at least one active pharmaceutical ingredient before, during or after granulation; and
preparing a pharmaceutical formulation in the form of a dosage form;
wherein the at least one filler is not a phosphate compound;
wherein the at least one binder is not a PVP compound; and
wherein the formulation has a bulk density greater than 0.5 g/mL.

**12.** The method of claim 11, wherein the pharmaceutical formulation further comprises 5-[3-(2,5-dichloro-4,6-dimethylpyridin-3-yl)-[1,2,4]oxadiazol-5-yl]-3-nitrobenzene-1,2-diol.

**13.** The method of claims 11 or 12 wherein:

a. the at least one filler is chosen from lactose, maize starch and microcrystalline cellulose; or
b. the at least one binder is chosen from hypromellose, hydroxypropyl cellulose, methyl- or ethyl-cellulose, pregelatinized maize starch and gelatin.

**14.** The method of any of claims 11 to 13, wherein:

a. the granulation is conducted in a high shear mixer or in a fluid bed dryer; or
b. the granulation process is wet granulation, further comprising drying the granules.

**15.** The method of any of claims 11 to 14, further comprising:

a. sieving the granules; or
b. adding at least one additional excipient before, during or after granulation.

**16.** The method of any of claims 11 to 15, wherein the dosage form is a tablet, and the step of preparing the formulation comprises compression.

**17.** The method of any of claims 11 to 16, wherein the dosage form is a capsule, and the step of preparing the formulation comprises filling a capsule.

**18.** The stable composition or pharmaceutical formulation of any one of claims 1 to 10, obtainable by the method of any of claims 11 to 17.

## Patentansprüche

**1.** Stabile Zusammensetzung, umfassend:

einen pharmazeutischen Wirkstoff (API) ausgewählt aus 2,5-Dichlor-3-(5-(3,4-dihydroxy-5-nitrophenyl)-1,2,4-oxadiazol-3-yl)-4,6-dimethylpyridin-1-oxid und Salzen, Estern, Hydraten und Solvaten davon;
zumindest einen Füllstoff; und
zumindest ein Bindemittel;
wobei der pharmazeutische Wirkstoff in Granulatform vorliegt;
wobei der zumindest eine Füllstoff keine Phosphatverbindung ist;
wobei das zumindest eine Bindemittel keine PVP-Verbindung ist; und
wobei die Zusammensetzung eine Schüttdichte von mehr als 0,5 g/ml aufweist.

**2.** Stabile Zusammensetzung nach Anspruch 1, ferner umfassend 5-[3-(2,5-Dichlor-4,6-dimethylpyridin-3-yl)-[1,2,4]oxadiazol-5-yl]-3-nitrobenzol-1,2-diol.

**3.** Stabile Zusammensetzung nach Anspruch 1 oder 2, wobei:

a. sich weniger als 10 % oder weniger als 3 % des API zersetzen:

i. über 15 Tage Lagerung bei 70 °C und unkontrollierter Luftfeuchtigkeit;
ii. über 6 Monate bei 40 °C und 75 % relativer Luftfeuchtigkeit; und/oder
iii. über 3 Jahre bei 60 % relativer Luftfeuchtigkeit und 30 °C oder 25 °C; und/oder

b. die Zunahme der Gesamtverunreinigungen weniger als 5 % oder weniger als 0,5 % ist:

i. über 15 Tage Lagerung bei 70 °C und unkontrollierter Luftfeuchtigkeit;
ii. über 6 Monate bei 40 °C und 75 % relativer Luftfeuchtigkeit; und/oder
iii. über 3 Jahre bei 60 % relativer Luftfeuchtigkeit und 30 °C oder 25 °C.

**4.** Stabile Zusammensetzung nach einem vorhergehenden Anspruch, wobei der zumindest eine Füllstoff ausgewählt ist aus Lactose, Maisstärke und mikrokristalliner Cellulose und wobei das zumindest eine Bindemittel ausgewählt ist aus Hypromellose, Hydroxypropylcellulose, Methyl- oder Ethylcellulose, vorgelatinierter Maisstärke und Gelatine.

**5.** Stabile Zusammensetzung nach einem vorhergehenden Anspruch:

a. wobei das Granulat ferner den zumindest einen Füllstoff und/oder das zumindest eine Bindemittel umfasst; oder
b. ferner umfassend zumindest einen zusätzlichen Hilfsstoff ausgewählt aus Sprengmitteln, Gleitmitteln und Schmiermitteln; oder
c. wobei die Zusammensetzung eine Schüttdichte von mehr als 0,6 g/ml aufweist; oder
d. wobei die Zusammensetzung eine scheinbare Dichte von mehr als 0,1 g/ml oder mehr als 0,5 g/ml aufweist.

**6.** Stabile Zusammensetzung nach Anspruch 5, wobei das Schmiermittel ausgewählt ist aus Glycerinmonostearat, Glycerylbehenat, Glycerylpalmitostearat, hydriertem Rizinusöl, hydriertem Pflanzenöl Typ I, mittelkettigen Triglyceriden, Calciumstearat, Magnesiumlaurylsulphat, Magnesiumstearat, Natriumlaurylsulphat, Natriumstearylfumarat, Stearinsäure und Zinkstearat, bevorzugt Calciumstearat, Magnesiumlaurylsulphat, Magnesiumstearat, Natriumlaurylsulphat, Natriumstearylfumarat, Stearinsäure und Zinkstearat.

**7.** Pharmazeutische Formulierung, umfassend die stabile Zusammensetzung nach einem vorhergehenden Anspruch.

**8.** Pharmazeutische Formulierung nach Anspruch 7, wobei

a. der pharmazeutische Wirkstoff in einer therapeutisch wirksamen Menge vorliegt; oder
b. die Zusammensetzung einen zusätzlichen API umfasst; oder
c. die Formulierung in der Dosierungsform einer Tablette oder Kapsel ist; oder
d. die pharmazeutische Formulierung stabil ist.

**9.** Pharmazeutische Formulierung nach Anspruch 7 oder Anspruch 8, wobei der API in einer Menge von 1 mg oder mehr, 2,5 mg oder mehr, 5 mg oder mehr, 10 mg oder mehr, 20 mg oder mehr, 40 mg oder mehr, 50 mg oder mehr oder 100 mg oder mehr vorliegt und wobei der zumindest eine API in Granulatform vorliegt.

**10.** Pharmazeutische Formulierung nach Anspruch 9, ferner umfassend Carbidopa oder Benserazid.

**11.** Verfahren zum Herstellen einer stabilen pharmazeutischen Formulierung, wobei das Verfahren Folgendes umfasst:

Granulieren eines pharmazeutischen Wirkstoffes (API) ausgewählt aus 2,5-Dichlor-3-(5-(3,4-dihydroxy-5-nitrophenyl)-1,2,4-oxadiazol-3-yl)-4,6-dimethylpyridin-1-oxid und Salzen davon, um Granulat zu bilden;
Mischen von zumindest einem Füllstoff mit dem zumindest einen pharmazeutischen Wirkstoff vor, während oder nach Granulierung;
Mischen von zumindest einem Bindemittel mit dem zumindest einen pharmazeutischen Wirkstoff vor, während oder nach Granulierung; und
Zubereiten einer pharmazeutischen Formulierung in der Form einer Dosierungsform;
wobei der zumindest eine Füllstoff keine Phosphatverbindung ist;
wobei das zumindest eine Bindemittel keine PVP-Verbindung ist; und
wobei die Formulierung eine Schüttdichte von mehr als 0,5 g/ml aufweist.

**12.** Verfahren nach Anspruch 11, wobei die pharmazeutische Formulierung ferner 5-[3-(2,5-Dichlor-4,6-dimethylpyridin-3-yl)-[1,2,4]oxadiazol-5-yl]-3-nitrobenzol-1,2-diol umfasst.

**13.** Verfahren nach Anspruch 11 oder 12, wobei:

a. der zumindest eine Füllstoff ausgewählt ist aus Lactose, Maisstärke und mikrokristalliner Cellulose; oder
b. das zumindest eine Bindemittel ausgewählt ist aus Hypromellose, Hydroxypropylcellulose, Methyl- oder Ethylcellulose, vorgelatinierter Maisstärke und Gelatine.

**14.** Verfahren nach einem der Ansprüche 11 bis 13, wobei:

a. die Granulierung in einem Hochschermischer oder in einem Wirbelschichttrockner durchgeführt wird; oder
b. der Granulierungsprozess Nassgranulation ist, ferner umfassend das Trocknen des Granulats.

**15.** Verfahren nach einem der Ansprüche 11 bis 14, ferner umfassend:

a. Sieben des Granulats; oder
b. Hinzufügen von zumindest einem zusätzlichen Hilfsstoff vor, während oder nach Granulierung.

**16.** Verfahren nach einem der Ansprüche 11 bis 15, wobei die Dosierungsform eine Tablette ist und der Schritt des Zubereitens der Formulierung Kompression umfasst.

**17.** Verfahren nach einem der Ansprüche 11 bis 16, wobei die Dosierungsform eine Kapsel ist und der Schritt des Zubereitens der Formulierung das Füllen einer Kapsel umfasst.

**18.** Stabile Zusammensetzung oder pharmazeutische Formulierung nach einem der Ansprüche 1 bis 10, erhältlich durch das Verfahren nach einem der Ansprüche 11 bis 17.

## Revendications

**1.** Composition stable comprenant :

un ingrédient pharmaceutique actif (IPA) choisi entre 2,5-dichloro-3-(5-(3,4-dihydroxy-5-nitrophényl)-1,2,4-oxadiazol-3-yl)-4,6-diméthylpyridine 1-oxyde et sels, esters, hydrates et solvates de celui-ci ;
au moins une charge ; et
au moins un liant ;
où l'ingrédient pharmaceutique actif est présent sous forme granulaire ;
où la ou les charges ne sont pas un composé de phosphate ;
où le ou les liants ne sont pas un composé de PVP ; et
où la composition a une masse volumique apparente supérieure à 0,5 g/mL.

**2.** Composition stable selon la revendication 1, comprenant en outre 5-[3-(2,5-dichloro-4,6-diméthylpyridin-3-yl)-[1,2,4]oxadiazol-5-yl]-3-nitrobenzène-1,2-diol.

**3.** Composition stable selon la revendication 1 ou 2, dans laquelle :

a. moins de 10 % ou moins de 3 % de l'IPA se décompose :

i. pendant 15 jours de stockage à 70°C et à une humidité non contrôlée ;
ii. pendant 6 mois à 40°C et à une humidité relative de 75 % ; et/ou
iii. pendant 3 ans à une humidité relative de 60 % et à 30°C ou 25°C ; et/ou

b. l'augmentation des impuretés totales est inférieure à 5 % ou inférieure à 0,5 % :

i. pendant 15 jours de stockage à 70°C et à une humidité non contrôlée ;
ii. pendant 6 mois à 40°C et à une humidité relative de 75 % ; et/ou
iii. pendant 3 ans à une humidité relative de 60 % et à 30°C ou 25°C.

**4.** Composition stable selon l'une quelconque des revendications précédentes, dans laquelle la ou les charges sont choisies entre : lactose, amidon de maïs et cellulose microcristalline, et dans laquelle le ou les liants sont choisis entre : hypromellose, hydroxypropylcellulose, méthyl- ou éthylcellulose, amidon de maïs pré-gélatinisé et gélatine.

**5.** Composition stable selon l'une quelconque des revendications précédentes :

a. dans laquelle les granules comprennent en outre la ou les charges et/ou au moins un liant ; ou
b. comprenant en outre au moins un excipient supplémentaire choisi entre : désagrégeants, glissants et lubrifiants ; ou
c. où la composition présente une masse volumique apparente supérieure à 0,6 g/ml ; ou
d. où la composition présente une densité apparente supérieure à 0,1 g/ml, ou supérieure à 0,5 g/ml.

**6.** Composition stable selon la revendication 5, dans laquelle le lubrifiant est sélectionné entre : monostéarate de glycérine, béhénate de glycéryle, palmitostéarate de glycéryle, huile de ricin hydrogénée, huile végétale hydrogénée type I, triglycérides à chaîne moyenne, stéarate de calcium, laurylsulfate de magnésium, stéarate de magnésium, laurylsulfate de sodium, stéarylfumarate de sodium, acide stéarique et stéarate de zinc, de préférence stéarate de calcium, laurylsulfate de magnésium, stéarate de magnésium, laurylsulfate de sodium, stéarylfumarate de sodium, acide stéarique et stéarate de zinc.

**7.** Formulation pharmaceutique comprenant la composition stable selon l'une quelconque des revendications précédentes.

**8.** Formulation pharmaceutique selon la revendication 7, dans laquelle

a. l'ingrédient pharmaceutique actif est présent en quantité ayant une efficacité thérapeutique ; ou
b. la composition comprend un IPA supplémentaire ; ou
c. la formulation a la forme médicamenteuse d'un comprimé ou d'une capsule ; ou
d. la formulation pharmaceutique est stable.

**9.** Formulation pharmaceutique selon la revendication 7 ou la revendication 8, dans laquelle l'IPA est présent à raison de 1 mg ou plus, 2,5 mg ou plus, 5 mg ou plus, 10 mg ou plus, 20 mg ou plus, 40 mg ou plus, 50 mg ou plus, ou 100 mg ou plus, et dans laquelle le ou les IPA sont présents sous forme granulaire.

**10.** Formulation pharmaceutique selon la revendication 9, comprenant en outre carbidopa ou bensérazide.

**11.** Procédé de fabrication d'une formulation pharmaceutique stable, ledit procédé comprenant :

la granulation d'un ingrédient pharmaceutique actif (IPA) choisi entre 2,5-dichloro-3-(5-(3,4-dihydroxy-5-nitrophényl)-1,2,4-oxadiazol-3-yl)-4,6-diméthylpyridine 1-oxyde et des sels de celui-ci pour former des granules ;
le mélange d'au moins une charge avec le ou les ingrédients pharmaceutiques actifs avant, pendant ou après la granulation ;
le mélange d'au moins un liant avec le ou les ingrédients pharmaceutiques actifs avant, pendant ou après la

granulation ; et
la préparation d'une formulation pharmaceutique sous la forme d'une forme médicamenteuse ;
dans lequel la ou les charges ne sont pas un composée de phosphate ;
dans lequel le ou les liants ne sont pas un composé de PVP ; et
dans lequel la formulation a une masse volumique apparente supérieure à 0,5 g/mL.

**12.** Procédé selon la revendication 11, dans lequel la formulation pharmaceutique comprend en outre 5-[3-(2,5-dichloro-4,6-diméthylpyridin-3-yl)-[1,2,4]oxadiazol-5-yl]-3-nitrobenzène-1,2-diol.

**13.** Procédé selon la revendication 11 ou 12, dans lequel :

a. la ou les charges sont choisies entre : lactose, amidon de maïs et cellulose microcristalline ; ou
b. le ou les liants sont choisis entre : hypromellose, hydroxypropylcellulose, méthyl-ou éthylcellulose, maïs prégélatinisé et gélatine.

**14.** Procédé selon l'une quelconque des revendications 11 à 13, dans lequel :

a. la granulation est effectuée dans un mélangeur à cisaillement élevé ou dans un séchoir à lit fluidisé ; ou
b. le processus de granulation est une granulation par voie humide, comprenant en outre le séchage des granules.

**15.** Procédé selon l'une quelconque des revendications 11 à 14, comprenant en outre :

a. le tamisage des granules ; ou
b. l'adjonction d'au moins un excipient supplémentaire avant, pendant ou après la granulation.

**16.** Procédé selon l'une quelconque des revendications 11 à 15, dans lequel la forme médicamenteuse est un comprimé, et l'étape de préparation de la formulation comprend la compression.

**17.** Procédé selon l'une quelconque des revendications 11 à 16, dans lequel la forme médicamenteuse est une capsule, et l'étape de préparation de la formulation comprend le remplissage d'une capsule.

**18.** Composition stable ou formulation pharmaceutique selon l'une quelconque des revendications 1 à 10, pouvant être obtenue par le procédé de l'une quelconque des revendications 11 à 17.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007013830 A
- WO 2007117165 A
- WO 2008094053 A1


### Non-patent literature cited in the description


- European Pharmacopeia. EDQM, 2007, 285-286
- *The United States Pharmacopeia Convention,* 2008, vol. 1, 231-232
- *The United States Pharmacopeia Convention,* 2008, vol. 1